Europäisches Patentamt

European Patent Office

Office européen des brevets

(19)

(11) Publication number : **0 592 835 A2**

# (12) EUROPEAN PATENT APPLICATION

(21) Application number : 93114929.8

(22) Date of filing : 16.09.93

(51) Int. Cl.[5] : **C12P 1/04,** C07G 11/00, C12N 1/20, // (C12N1/20, C12R1:01), (C12P1/04, C12R1:01)

The microorganism(s) has (have) been deposited with American Type Culture Collection under number(s) ATCC 55327.

(30) Priority : **23.09.92 US 950106**

(43) Date of publication of application : **20.04.94 Bulletin 94/16**

(84) Designated Contracting States : **AT BE CH DE DK ES FR GB GR IE IT LI LU MC NL PT SE**

(71) Applicant : **Bristol-Myers Squibb Company 345 Park Avenue New York, N.Y. 10154 (US)**

(72) Inventor : **Nishio, Maki 2-10-2, Nishikamata Ohta-ku, Tokyo (JP)**
Inventor : **Nihei, Yoshimi Mezon-Entopia A-202, 356-1, Iizuka-cho Takasaki, Gunma 370 (JP)**
Inventor : **Suzuki, Kiyoshi 3265 Noborito, Tama-ku Kawasaki, Kanagawa (JP)**
Inventor : **Hanada, Minoru 7-7-9, Nishiogotanda Shinagawa-ku, Tokyo (JP)**

(74) Representative : **Josif, Albert, Dr.-Ing. et al Baaderstrasse 3 D-80469 München (DE)**

(54) **BU-4803T Antibiotics.**

(57)   The present invention relates to antitumor antibiotics designated as BU-4803T $A_1$, $A_2$, B, $C_1$, $C_2$ and D. BU-4803T $A_1$, $A_2$ and B are produced by fermentation of <u>Microbispora</u> strain AA9966 which has been deposited with the American Type Culture Collection under the accession number ATCC 55327.

FIG.1

## BACKGROUND OF THE INVENTION

The present invention relates to novel antitumor compounds, the production by fermentation process thereof, and the producing microorganism Microbispora strain AA9966.

## SUMMARY OF THE INVENTION

The present invention provides novel antibiotic compounds herein designated as BU-4803T $A_1$, $A_2$, B, $C_1$, $C_2$ and D having the physico-chemical properties listed hereinafter. These compounds are useful as antitumor agents; additionally, BU-4803T $A_1$, $A_2$ and B are active against Gram-positive bacteria.

Another aspect of the invention provides a process for the production of BU-4803T $A_1$, $A_2$ and B which comprises cultivating an antibiotic producing strain of Microbispora sp. in an aqueous medium containing assimilable sources of carbon and nitrogen under aerobic conditions, and recovering said antibiotic from the fermentation broth.

A further aspect of the present invention provides a biologically pure culture of a BU-4803T producing strain of Microbispora sp. strain AA9966 having the identifying characteristics of ATCC 55327.

## BRIEF DESCRIPTION OF THE DRAWINGS

Figure 1 shows an IR spectrum of BU-4803T $A_1$.
Figure 2 shows an IR spectrum of BU-4803T $A_2$.
Figure 3 shows an IR spectrum of BU-4803T B.
Figure 4 shows an IR spectrum of BU-4803T $C_1$.
Figure 5 shows an IR spectrum of BU-4803T $C_2$.
Figure 6 shows an IR spectrum of BU-4803T D.
Figure 7 shows a proton NMR spectrum of BU-4803T $A_1$ (400 MHz, $CDCl_3$).
Figure 8 shows a proton NMR spectrum of BU-4803T $A_2$ (400 MHz, $CDCl_3$).
Figure 9 shows a proton NMR spectrum of BU-4803T B (400 MHz, $CDCl_3$).
Figure 10 shows a proton NMR spectrum of BU-4803T $C_1$ (400 MHz, $CDCl_3$).
Figure 11 shows a proton NMR-spectrum of BU-4803T $C_2$ (400 MHz, $CDCl_3$).

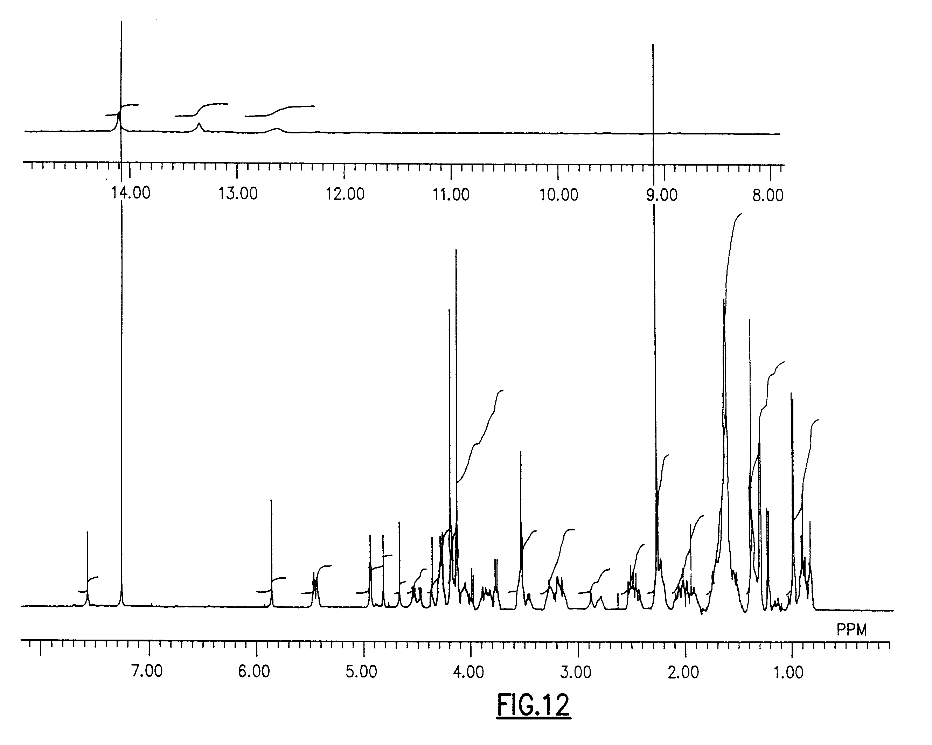

Figure 12 shows a proton NMR spectrum of BU-4803 D (400 MHz, $CDCl_3$).

## DETAILED DESCRIPTION OF THE INVENTION

The physico-chemical properties of antibiotic BU-4803T $A_1$, $A_2$, B, $C_1$, $C_2$ and b are given below. The optical rotations of the compounds have not been determined because of strong UV absorption at 589 nm. Melting points are determined with a Yanaco micro melting point apparatus and are uncorrected. UV and IR spectra are recorded on a Jasco UVIDEC-610C spectrometer and a Jasco IR-810 spectrometer, respectively. The $^1$H and $^{13}$C NMR spectra are recorded on a Jeol JMN-GX 400 spectrometer with $CDCl_3$ as an internal standard. FAB-MS are run with a Jeol JMS-AX505H spectrometer using 3-nitrobenzyl alcohol as a matrix. Elemental analysis are measured on Perkin-Elmer 240C Elemental Analyzer. The HPLC is determined using a Cosmosil 5C18 AR column (4.6 mm i.d. x 150 mm, Nacalai Tesque, Inc.); $CH_3CN-H_2O$ (43:57) as the mobile phase at a flow rate of 1.2 ml/min.; and UV absorption at 254 nm as the detection means. The TLC is determined on a RP-18 $F_{254S}$ plate (Merck, Art 15423) using $MeOH-H_2O$ (87.5:12.5) as the solvent system.

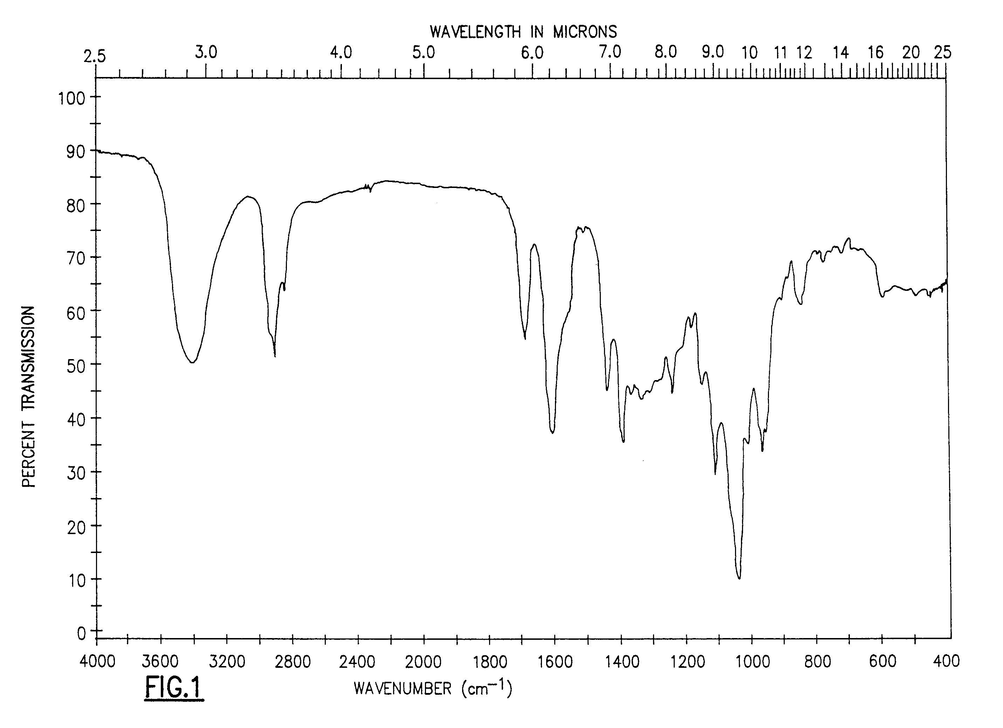

BU-4803T $A_1$
Nature: red powder
M.P. (°C, dec.): >200
Solubility:
    Soluble in: Dimethyl sulfoxide, methanol, acetonitrile, acetone, and methylene chloride
    Insoluble in: Hexane and water
UV $\lambda_{max}$ nm ($E_{1cm}^{1\%}$):
    in MeOH: 244 (222), 278 (267), 433 (92), 509 (58)
    in 0.1 N HCl-MeOH (1:9): 239 (277), 281 (291), 433 (78), 510 (65)
    in 0.1N NaOH-MeOH (1:9): 236 (217), 278 (292), 437 (119), 613 (51), 648 (50)
IR $\nu_{max}$ (KBr) cm$^{-1}$: 3450, 2940, 1700, 1620, 1455, 1405, 1120, 1055 (as shown in Figure 1).
FAB-MS m/z:
    Positive: 1764 (M+H+Na)$^+$

Negative: 1741 (M+2H-H)⁻

Elemental Analysis: C 58.20%; H 6.70%; N <0.3%

HPLC Rt (min.): 10.4

TLC Rf: 0.49

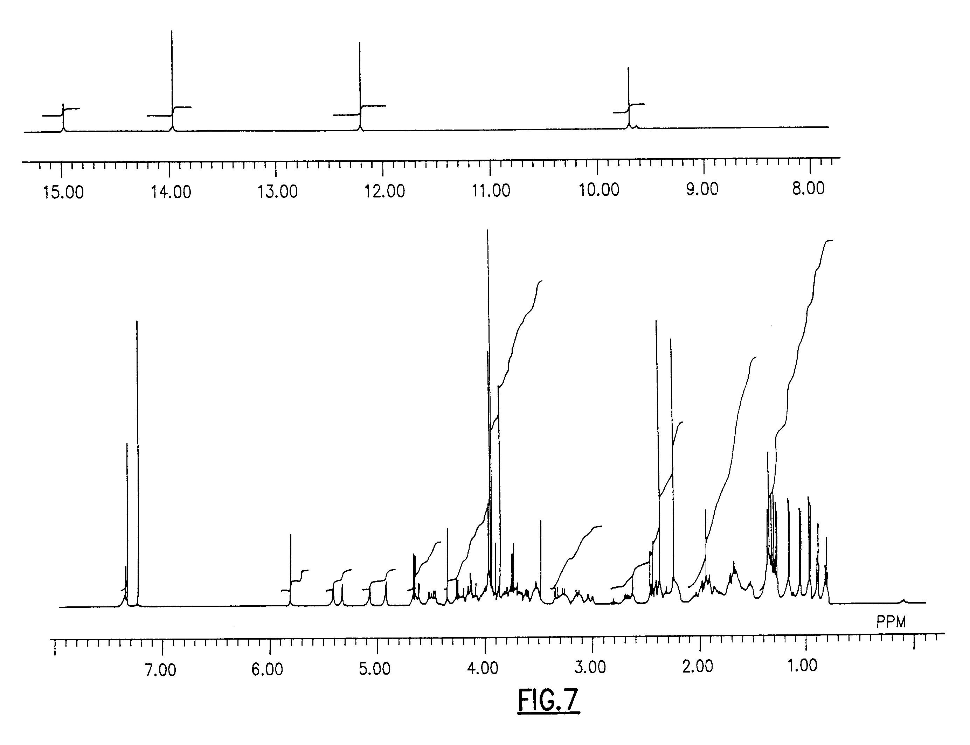

$^1$H-NMR (CDCl$_3$): 0.79 (3H, t), 0.87 (3H, t), 0.95 (3H, d), 1.00 (1H, m), 1.04 (3H, d), 1.10 (1H, m), 1.15 (3H, d), 1.29 (3H, d), 1.30 (1H, m), 1.33 (3H, d), 1.34 (2H, m), 1.35 (3H, d), 1.41-1.94 (13H), 1.93 (1H, dd), 1.94 (1H), 1.97 (1H, dd), 2.04 (1H, dt), 2.22 (1H, dt), 2.24-2.38 (2H), 2.24 (3H, s), 2.38 (3H, s), 2.39 (1H, dd), 2.43 (1H, dd), 2.44 (1H), 2.47 (1H, d), 2.64 (1H, d), 2.69 (1H, qui), 3.03 (1H, dd), 3.12 (1H, dt), 3.16 (1H, dt), 3.24-3.33 (2H, dt), 3.34 (1H, d), 3.49 (1H), 3.50-3.60 (2H, dt), 3.64 (1H, dt), 3.68 (1H, dd), 3.72 (1H), 3.73-3.76 (2H), 3.74 (1H), 3.76 (1H), 3.79-3.82 (1H), 3.87 (3H, s), 3.91 (1H, s), 3.95 (3H, s), 3.97 (3H, s), 3.98 (1H), 4.01 (2H, dd), 4.06-4.13 (2H), 4.16 (2H, dd), 4.21 (1H), 4.27 (1H, dd), 4.36 (1H), 4.48 (1H, dd), 4.52 (1H, dd), 4.62 (1H, dd), 4.66 (1H, s), 4.68 (1H, s), 4.94 (1H, d), 5.09 (1H, d), 5.35 (1H, d), 5.43 (1H, d), 5.83 (1H, s), 7.38 (1H, s), 9.69 (1H, s), 12.21 (1H, s), 13.95 (1H, s), 14.96 (1H, s) (as shown in Figure 7)

## BU-4803T A₂

Nature: red powder

M.P. (°C, dec.): >200

Solubility:

Soluble in: Dimethyl sulfoxide, methanol, acetonitrile, acetone, and methylene chloride

Insoluble in: Hexane and water

UV $\lambda_{max}$ nm ($E_{1cm}^{1\%}$):

in MeOH: 240 (261), 279 (276), 432 (86), 510 (68)

in 0.1 N HCl-MeOH (1:9): 239 (275), 280 (288), 432 (76), 509 (70)

in 0.1N NaOH-MeOH (1:9): 235 (214), 278 (279), 436 (113), 615 (54), 647 (54)

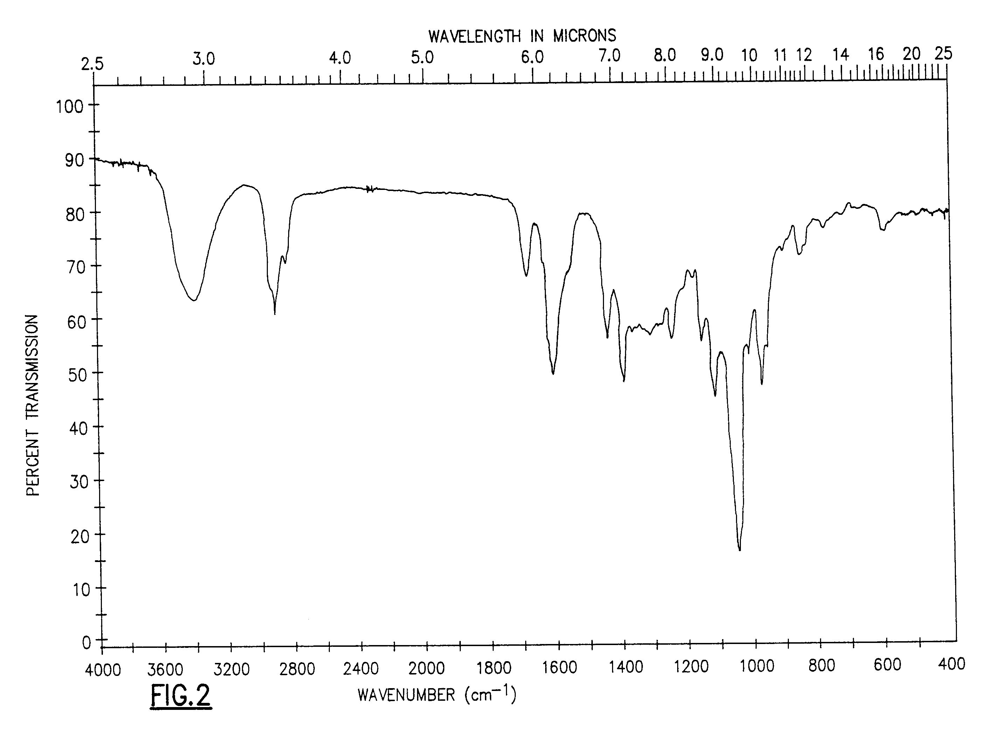

IR $\nu_{max}$ (KBr) cm⁻¹: 3450, 2940, 1700, 1620, 1455, 1405, 1120, 1055 (as shown in Figure 2)

FAB-MS m/z:

Positive: 1705 (M+Na)⁺

Negative: 1683 (M+2H-H)⁻

HPLC Rt (min.): 11.1

TLC Rf: 0.43

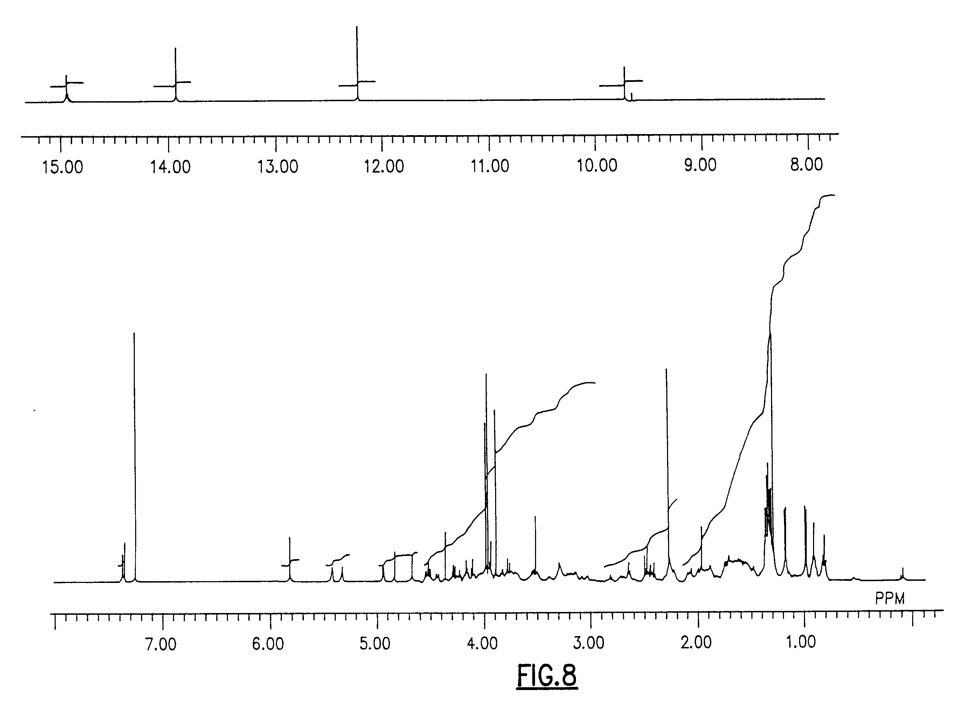

$^1$H-NMR (CDCl$_3$): 0.78 (3H, t), 0.87 (3H, t), 0.95 (3H, d), 0.99 (1H, m), 1.05 (1H, m), 1.14 (3H, d), 1.25-1.34 (5H), 1.29 (3H, d), 1.30 (3H, d), 1.31 (3H, d), 1.44-2.07 (15H), 1.93 (1H), 1.98 (1H), 2.00 (1H), 2.23 (2H), 2.24 (3H, s), 2.26 (1H), 2.39 (1H, dd), 2.43 (1H, dd), 2.44 (1H), 2.47 (1H), 2.63 (1H, d), 2.69 (1H, qui), 3.03 (1H, dd), 3.12 (1H, dt), 3.16 (1H, dt), 3.20-3.30 (3H), 3.36 (1H, d), 3.49 (1H), 3.47-3.55 (2H), 3.68 (1H, dd), 3.72 (1H), 3.74-3.76 (2H), 3.80 (1H), 3.79-3.82 (1H), 3.87 (3H, s), 3.91 (1H, s), 3.93 (1H, s), 3.95 (3H, s), 3.97 (3H, s), 3.99 (1H), 4.02 (1H), 4.02 (1H), 4.06-4.10 (1H), 4.13-4.17 (2H), 4.21 (1H), 4.27 (1H), 4.35 (1H), 4.42 (1H), 4.49 (1H, dd), 4.52 (1H, d), 4.66 (1H), 4.82 (1H), 4.93 (1H, d), 5.34 (1H, d), 5.43 (1H, d), 5.83 (1H, s), 7.38 (1H, s), 9.69 (1H, s), 12.20 (1H, s), 13.94 (1H, s), 14.96 (1H, s) (as shown in Figure 8)

## BU-4803T B

Nature: red powder

M.P. (°C, dec.): >200

Solubility:

Soluble in: Dimethyl sulfoxide, methanol, acetonitrile, acetone, methylene chloride

Insoluble in: Hexane and water

UV $\lambda_{max}$ nm ($E_{1cm}^{1\%}$):

in MeOH: 240 (239), 278 (254), 432 (80), 511 (62)

in 0.1 N HCl-MeOH (1:9): 239 (260), 281 (271), 431 (71), 510 (67)

in 0.1N NaOH-MeOH (1:9): 235 (211), 278 (272), 436 (111), 614 (51), 647 (51)

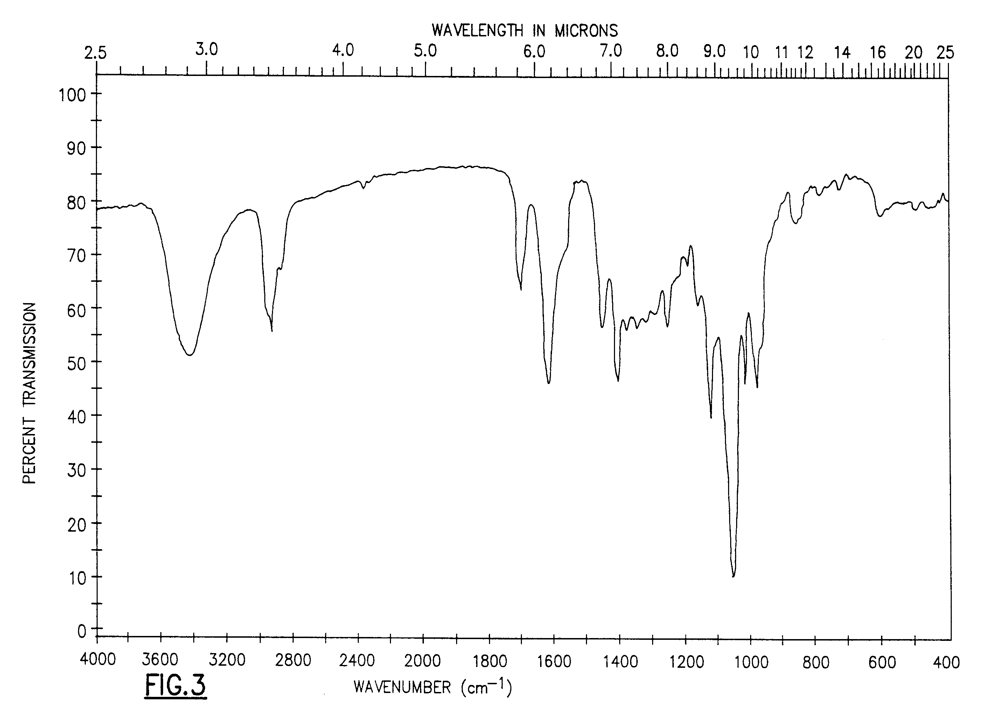

IR $\nu_{max}$ (KBr) cm⁻¹: 3450, 2940, 1705, 1620, 1460, 1405, 1125, 1060 (as shown in Figure 3)

FAB-MS m/z:

Positive: 1749 (M+2H+Na)⁺

Negative: 1725 (M+2H-H)⁻

Elemental Analysis: C 58.45%; H 6.48%; N <0.3%

HPLC Rt (min.): 18.0

TLC Rf: 0.39

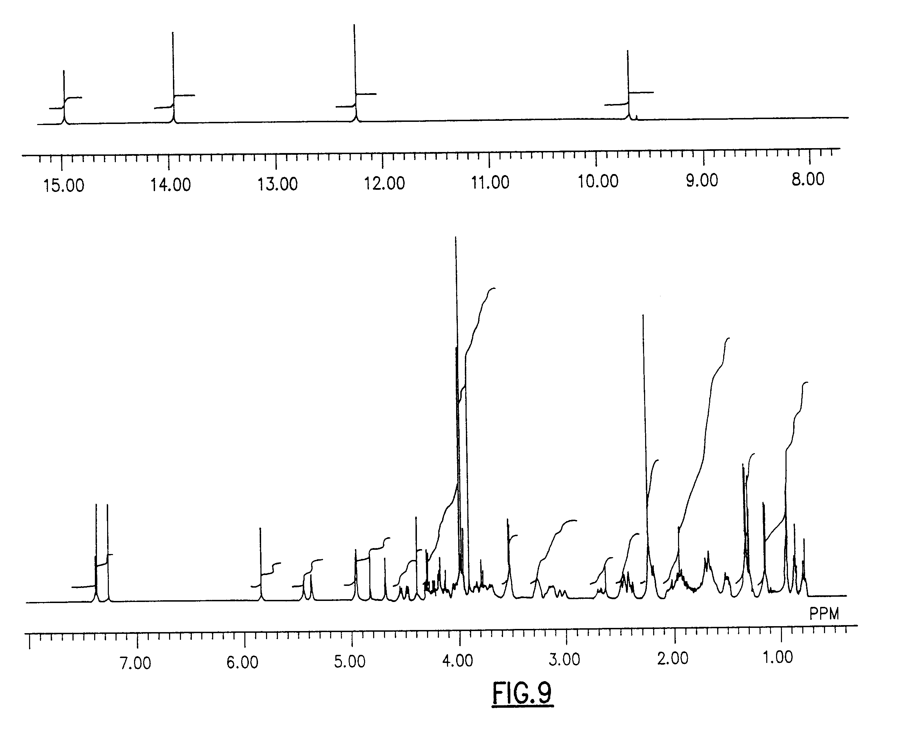

$^1$H-NMR (CDCl$_3$): 0.77 (3H, t), 0.85 (3H, t), 0.92 (3H, d), 0.93 (3H, d), 0.97 (1H, m), 1.06 (1H, dt), 1.12 (3H, d), 1.27 (3H, d), 1.30 (1H), 1.31 (3H, d), 1.31 (3H, d), 1.32 (2H), 1.45-1.66 (2H), 1.48 (2H), 1.62 (2H), 1.62-1.96 (4H), 1.66 (2H), 1.88 (1H, dt), 1.92 (1H, dd), 1.93 (1H, dd), 1.98 (2H, m), 1.98 (1H, s), 2.18 (2H), 2.20 (2H), 2.22 (3H, s), 2.23 (3H, s), 2.38 (1H, dd), 2.41 (1H, dd), 2.49 (1H), 2.52 (1H), 2.63 (1H, d), 2.67 (1H, qui), 3.02 (1H, dd), 3.10 (1H, dd), 3.13 (1H, dd), 3.24 (2H, dt), 3.37 (1H, d), 3.47-3.56 (2H), 3.54 (1H, s), 3.55 (1H, s), 3.66 (1H, m), 3.73 (1H, m), 3.74 (1H), 3.80 (1H, m), 3.85 (3H, s), 3.93 (3H, s), 3.95 (1H), 3.95 (3H, s), 3.96 (1H, d), 4.00 (1H, d), 4.08-4.23 (3H, m), 4.25 (1H, dt), 4.25 (1H, dt), 4.37 (1H, s), 4.45 (1H, d), 4.51 (1H, d), 4.66 (1H, s), 4.80 (1H, s), 4.92 (1H, d), 4.92 (1H, d), 5.32 (1H, d), 5.40 (1H, d), 5.81 (1H, s), 7.37 (1H, s), 9.69 (1H, s), 12.20 (1H, s), 13.94 (1H, s), 14.96 (1H, s) (as shown in Figure 9)

$^{13}$C-NMR (CDCl$_3$): 210.6 s, 210.6 s, 203.5 s, 195.5 s, 187.8 s, 184.8 s, 163.6 s, 158.3 s, 156.8 s, 153.2 s, 151.8 s, 150.6 s, 147.7 s, 139.4 s, 138.5 s, 135.4 s, 125.6 s, 124.7 s, 116.3 s, 113.0 s, 112.1 d, 110.5 s, 108.2 s, 107.9 s, 103.2 d, 103.2 d, 98.9 d, 98.9 d, 98.9 d, 98.7 d, 86.6 d, 85.6 s, 85.4 d, 82.7 s, 79.3 s, 78.9 d, 78.8 d, 78.5 s, 77.2 s, 77.2 s, 77.0 d, 76.3 d, 75.4 d, 75.3 d, 75.2 d, 72.7 d, 72.5 d, 70.6 d, 67.8 d, 67.1 d, 67.0 d, 66.7 d, 66.7 d, 65.1 d, 65.0 d, 61.1 q, 60.9 q, 60.8 q, 55.7 d, 44.4 d, 37.0 t, 35.3 t, 34.8 t, 34.3 t, 30.5 t, 30.4 t, 29.5 t, 29.4 t, 27.8 t, 27.8 t, 27.8 t, 25.0 q, 25.0 q, 24.8 t, 24.8 t, 18.1 q, 18.1 q, 18.0 t, 17.9 q, 17.7 q, 16.6 t, 15.1 q, 14.9 q, 14.6 q, 14.6 q

BU-4803T C$_1$

Nature: purple powder

M.P. (°C, dec.): >200

Solubility:

    Soluble in: Dimethyl sulfoxide and methylene chloride

    Slightly soluble in: Methanol and acetonitrile

    Insoluble in: Acetone, hexane and water

UV $\lambda_{max}$ nm ($E_{1cm}^{1\%}$):

    in MeOH: 260 (237), 291 (382), 392 (45), 547 (109)

    in 0.1 N HCl-MeOH (1:9): 260 (237), 291 (382), 391 (45), 546 (115)

    in 0.1N NaOH-MeOH (1:9): 292 (238), 457 (117), 482 (130), 679 (61)

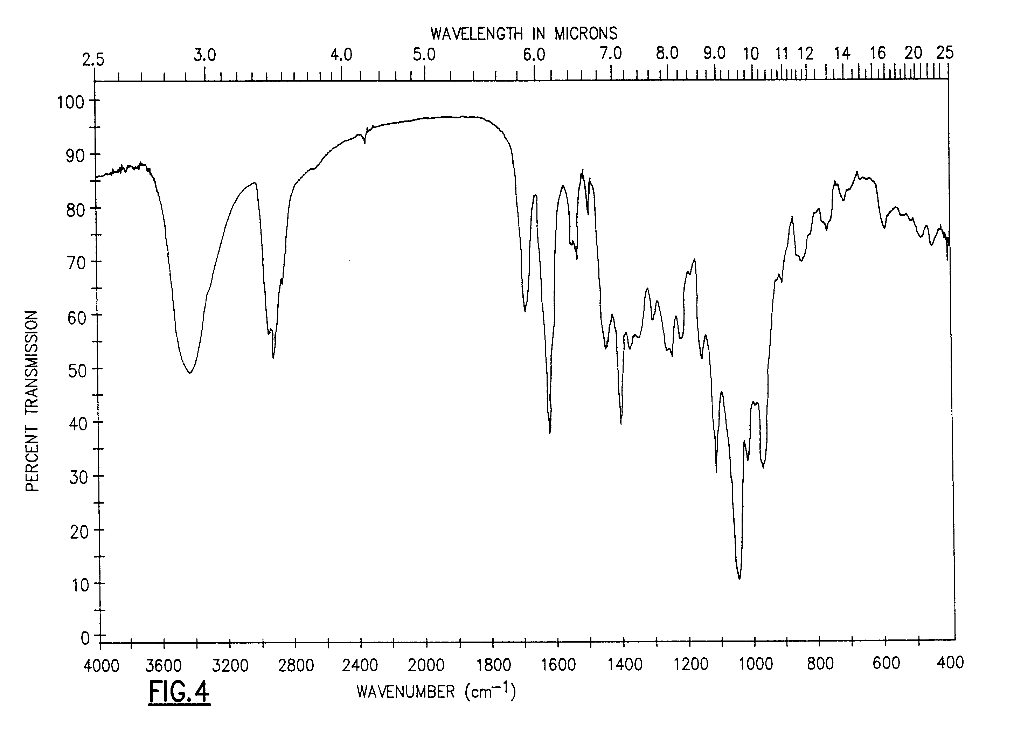

IR $\nu_{max}$ (KBr) cm$^{-1}$: 3450, 2940, 1700, 1625, 1455, 1410, 1120, 1055 (as shown in Figure 4)

FAB-MS m/z:

    Positive: 1732 (M+H+Na)$^+$

    Negative: 1709 (M+2H-H)$^-$

Elemental Analysis: C 57.27%; H 6.27%; N <0.3%

HPLC Rt (min.): 9.7

TLC Rf: 0.38

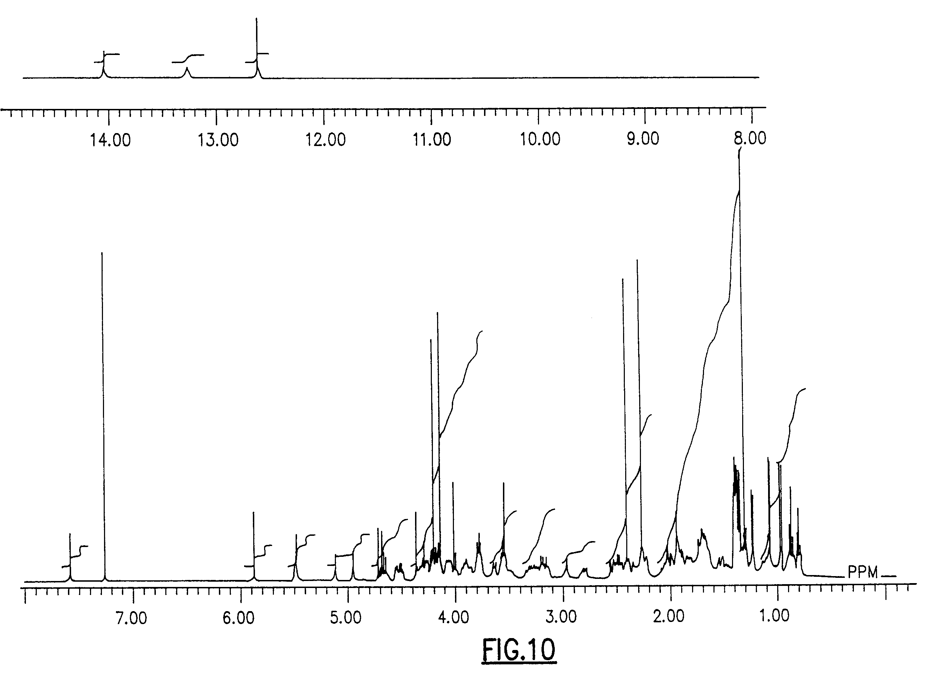

$^1$H-NMR (CDCl$_3$): 0.76 (3H, t), 0.83 (3H, t), 0.93 (3H, d), 0.98 (1H, m), 1.04 (3H, d), 1.07 (1H, m), 1.19 (3H, d), 1.25-1.38 (5H), 1.33 (3H, d), 1.36 (3H, d), 1.36 (3H, d), 1.38-2.08 (18H), 1.91 (1H), 1.97 (1H), 2.02 (1H), 2.15-2.38 (4H), 2.24 (3H, s), 2.38 (3H, s), 2.43 (1H, dd), 2.46 (1H, dd), 2.49 (1H, d), 2.53 (1H, d), 2.79 (1H, qui), 2.95 (1H, d), 3.11-3.24 (3H), 3.24 (1H), 3.30 (1H), 3.52 (1H), 3.52 (1H), 3.54 (1H), 3.62 (1H, dt), 3.75 (1H, d), 3.76 (1H), 3.78-3.90 (3H), 3.98 (1H, d), 3.99 (1H), 4.02 (1H), 4.05 (1H), 4.10-4.22 (3H), 4.12 (3H, s), 4.18 (3H, s), 4.22-4.33 (3H), 4.34 (1H), 4.49 (1H, dd), 4.53 (1H, dd), 4.62 (1H, dd), 4.66 (1H, s), 4.69 (1H, s), 4.93 (1H, d), 5.09 (1H, d), 5.47 (2H, t), 5.86 (1H, s), 7.59 (1H, s), 12.61 (1H, s), 13.27 (1H, s), 14.05 (1H, s) (as shown in Figure 10)

BU-4803T C$_2$

Nature: purple powder

M.P. (°C, dec.): >200

Solubility:

    Soluble in: Dimethyl sulfoxide and methylene chloride

    Slightly soluble in: Methanol and acetonitrile

    Insoluble in: Acetone, hexane and water

UV $\lambda_{max}$ nm ($E_{1cm}^{1\%}$):

    in MeOH: 261 (257), 292 (392), 394 (47), 552 (102)

    in 0.1 N HCl-MeOH (1:9): 260 (279), 291 (427), 392 (51), 548 (123)

    in 0.1N NaOH-MeOH (1:9): 292 (302), 457 (139), 482 (151), 676 (74)

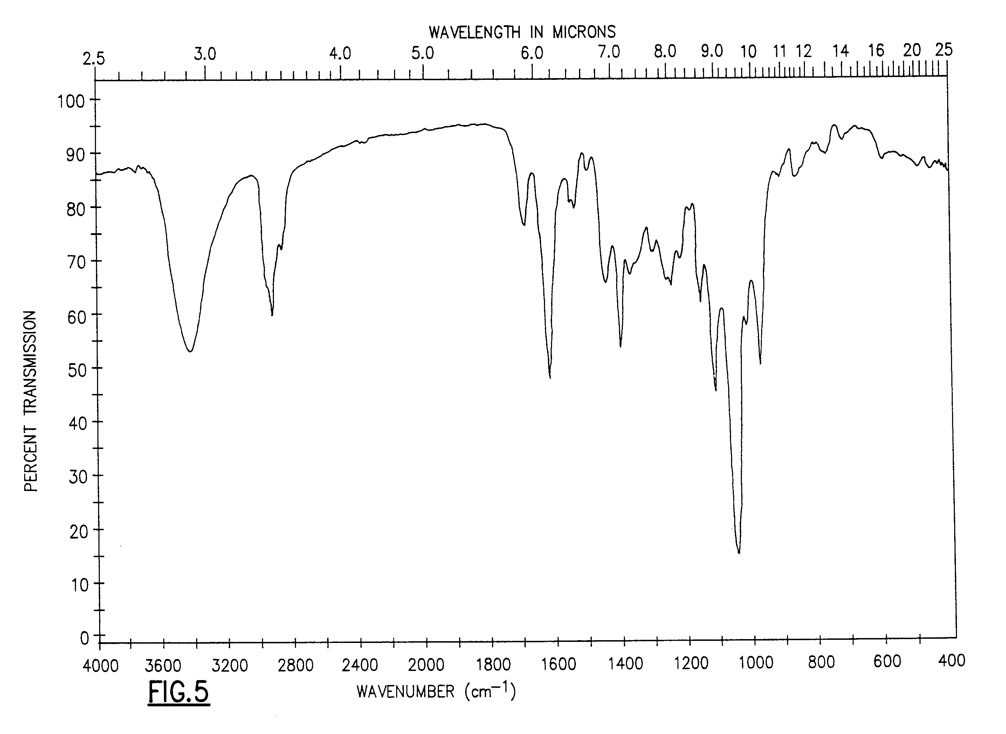

IR $\nu_{max}$ (KBr) cm$^{-1}$: 3450, 2940, 1695, 1625, 1455, 1410, 1125, 1055 (as shown in Figure 5)

FAB-MS m/z:

    Positive: 1674 (M+H+Na)$^+$

    Negative: 1651 (M+2H-H)$^-$

HPLC Rt (min.): 10.8

TLC Rf: 0.30

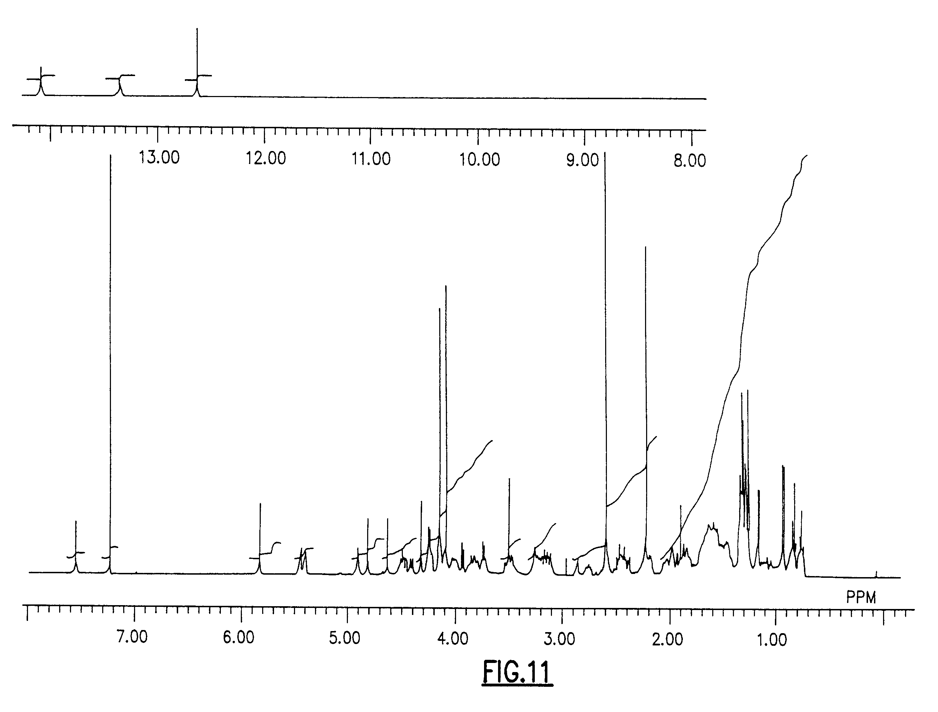

$^1$H-NMR (CDCl$_3$): 0.76 (3H, t), 0.84 (3H, t), 0.93 (3H, d), 0.99 (1H, m), 1.09 (1H, m), 1.17 (3H, d), 1.25-1.38 (5H), 1.33 (3H, s), 1.33 (3H, s), 1.34 (3H, s), 1.42-2.08 (17H), 1.92 (1H), 2.15-2.30 (4H), 2.24 (3H, s), 2.41 (1H, dd), 2.46 (1H, dd), 2.49 (1H, d); 2.52 (1H, d), 2.62 (6H, s), 2.78 (1H, qui), 2.88 (1H, d), 3.09-3.30 (6H), 3.49 (1H, dt), 3.52 (1H), 3.53 (1H, dt), 3.75 (1H, d), 3.78-3.86 (3H), 3.95 (1H, d), 4.01 (1H, d), 4.04 (1H, d), 4.10-4.20 (3H), 4.11 (3H, s), 4.17 (3H, s), 4.24-4.30 (3H), 4.34 (1H), 4.43 (1H, dd), 4.50 (1H, d), 4.53 (1H), 4.66 (1H), 4.84 (1H), 4.93 (1H, d), 5.42 (1H, d), 5.46 (1H, d), 5.86 (1H, s), 7.58 (1H, s), 12.63 (1H, s), 13.36 (1H, s), 14.09 (1H, s) (as shown in Figure 11)

BU-4803T D

Nature: purple powder

M.P. (°C, dec.): >200

Solubility:

Soluble in: Dimethyl sulfoxide and methylene chloride

Slightly soluble in: Methanol and acetonitrile

Insoluble in: Acetone, hexane and water

UV $\lambda_{max}$ nm ($E_{1cm}^{1\%}$):

in MeOH: 261 (217), 291 (347), 395 (39), 548 (97)

in 0.1 N HCl-MeOH (1:9): 260 (243), 291 (383), 392 (45), 545 (113)

in 0.1N NaOH-MeOH (1:9): 292 (234), 457 (113), 482 (126), 677 (60)

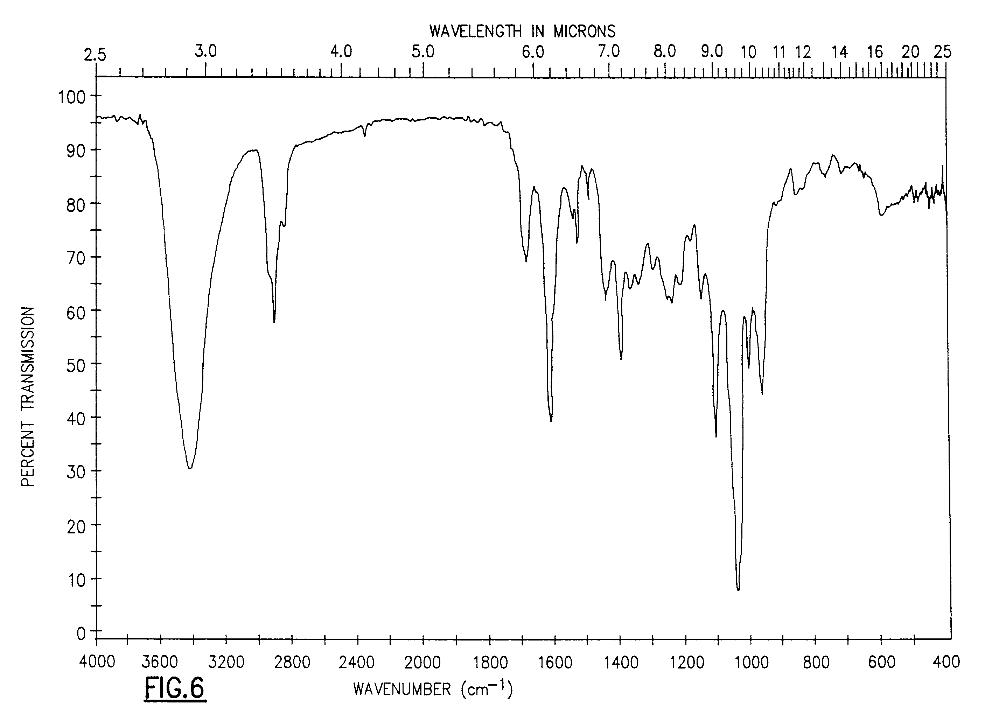

IR $\nu_{max}$ (KBr) cm$^{-1}$: 3450, 2940, 1700, 1625, 1455, 1410, 1125, 1055 (as shown in Figure 6)

FAB-MS m/z:

Positive: 1717 $(M+2H+Na)^+$

Negative: 1693 $(M+2H-H)^-$

Elemental Analysis: C 58.32%; H 6.34%; N <0.3%

HPLC Rt (min.): 17.0

TLC Rf: 0.25

$^1$H-NMR (CDCl$_3$): 0.77 (3H, t), 0.84 (3H, t), 0.93 (3H, d), 0.94 (3H, d), 0.99 (1H), 1.07 (1H, m), 1.17 (3H, d), 1.24-1.36 (4H), 1.33 (3H, d), 1.33 (3H, d), 1.34 (3H, d), 1.45-1.86 (6H), 1.88-2.06 (5H), 1.92 (1H), 2.19 (2H), 2.24 (2H), 2.24 (3H, s), 2.24 (3H, s), 2.42 (1H, dd), 2.46 (1H, dd), 2.46 (1H, d), 2.51 (1H, d), 2.78 (1H, qui), 2.88 (1H, brs), 3.11 (1H), 3.13 (1H), 3.18 (1H, dd), 3.25 (2H, m), 3.45 (1H, d), 3.51 (2H), 3.52 (1H, dd), 3.54 (1H, dd), 3.75 (1H, d), 3.76 (1H), 3.78-3.90 (3H), 3.97 (1H, d), 4.01 (1H, d), 4.04 (1H, d), 4.10-4.20 (2H), 4.11 (3H, s), 4.17 (3H, s), 4.24-4.30 (4H), 4.35 (1H), 4.47 (1H, dd), 4.53 (1H, dd), 4.66 (1H), 4.81 (1H), 4.93 (1H, d), 4.93 (1H, d), 5.43 (1H, d), 5.47 (1H, d), 5.86 (1H, s), 7.58 (1H, s), 12.62 (1H, s), 13.37 (1H, s), 14.11 (1H, s)

$^{13}$C-NMR (CDCl$_3$): 210.5 s, 210.5 s, 203.7 s, 195.3 s, 183.2 s, 176.1 s, 161.2 s, 158.6 s, 152.2 s, 152.1 s, 151.6 s, 150.2 s, 147.6 s, 145.8 s, 143.2 s, 136.4 s, 125.5 s, 125.3 s, 116.3 s, 116.2 s, 113.7 s, 112.6 d, 112.8 s, 107.5 s, 103.3 d, 103.3 d, 99.0 d, 99.0 d, 98.9 d, 98.4 d, 86.8 d, 86.0 s, 85.7 d, 82.9 s, 79.4 s, 78.9 d, 78.8 d, 78.6 s, 77.2 s, 77.2 s, 76.7 d, 75.8 d, 75.4 d, 75.2 d, 75.0 d, 72.9 d, 72.7 d, 70.8 d, 67.9 d, 67.4 d, 67.4 d, 66.7 d, 66.7 d, 65.4 d, 65.1 d, 62.2 q, 61.6 q, 55.4 d, 44.0 d, 36.7 t, 35.3 t, 34.9 t, 34.1 t, 30.5 t, 30.4 t, 29.5 t, 29.4 t, 28.5 t, 27.8 t, 27.8 t, 25.0 q, 25.0 q, 24.9 t, 24.9 t, 18.1 q, 18.1 q, 18.1 q, 17.8 q, 17.7 t, 16.6 t, 15.1 q, 14.8 q, 14.6 q, 14.6 q

BU-4803T A$_1$, A$_2$ and B are produced by fermentation of <u>Microbispora</u> sp. strain AA9966 (ATCC 55327); BU-4803T C$_1$, C$_2$ and D are prepared from BU-4803T A$_1$, A$_2$ and B, respectively.

<u>Producing Organism</u>

An actinomycete strain AA9966 which produces new antitumor antibiotics, BU-4803T A$_1$, A$_2$ and B, has been isolated from a soil sample collected at Kitakoma-gunn, Yamanashi prefecture, Japan. A biologically pure culture of strain AA9966 identified as <u>Microbispora</u> sp. has been deposited with the American Type Culture Collection (ATCC) under the BUDAPEST TREATY ON THE INTERNATIONAL RECOGNITION OF THE DEPOSIT OF MICROORGANISMS FOR THE PURPOSES OF PATENT PROCEDURE, and all restrictions on the availability to the public of the deposited microorganism will be irrevocably removed upon the granting of a patent from this application. The deposited culture has been assigned the accession number ATCC S5327.

1. <u>Morphology.</u>

Strain AA9966 is cultivated on oatmeal agar (ISP No. 3) at 28 °C for 21 days. Under light microscope, it is observed that characteristic longitudinal pair spores are formed on aerial mycelium, but not on substrate mycelium. Under scanning electron microscope, it is observed that the spores are oval in shape, 1.5 x 1.2 µm in size, and have smooth surface.

## 2. Cultural and Physiological Characteristics.

Cultural and physiological characteristics are determined by cultivating strain AA9966 at 28 °C for 14 to 21 days on various media described by: 1) E.B. Shirling and D. Gottlieb in "Methods for Characterization of *Streptomyces* species," Intern. J. Syst. Bact. 16:313-340, 1966; 2) S.A. Waksman in The Actinomycetes. Vol. II. Classification, Identification and Description of Genera and Species, pp. 328-334, The Williams & Wilkins Co., Baltimore, 1961; 3) T. Arai in Culture Media for Actinomycetes (The Society for Actinomycetes Japan, 1975); and 4) G.F. Gauze, T.P. Preobrazhenskaya, E.S. Kudrina, N.O. Blinov, I.D. Ryabova and M.A. Sveshnikova in Problems in the Classification of Antagonistic Actinomycetes. State Publishing House for Medical Literature (in Russian), Medzig, Moscow, 1957. Color of mycelia and soluble pigments are assigned using the Manual of Color Names (Japan Color Enterprise Co., Ltd., 1987). Temperature range for growth is determined on yeast starch agar using a temperature gradient incubator TN-3 (Toyo Kagaku Sangyo Co., Ltd.).

Strain AA9966 grows relatively well on various media. The aerial mycelium is visible on all media except for glycerol nitrate agar, ISP-4 and -7 and nutrient agar. Table 1 shows the results obtained after Strain AA9966 has been cultivated at 28 °C for 21 days.

Aerial mass color is white to pale yellowish pink. Vegetative mycelium and reverse side of colony are yellowish white to dark yellowish brown. Light grayish blue soluble pigment is observed in oatmeal agar (ISP-3) and oatmeal-yeast extract agar.

The physiological characteristics of, and the utilization of carbon sources by, strain AA9966 are shown in Tables 2 and 3, respectively.

Table 1. Cultural characteristics of strain AA9966

| Medium | | |
|---|---|---|
| Sucrose nitrate agar (Waksman med. No. 1) | Vegetative mycelium | Yellowish white (393) |
| | Reverse | Yellowish white (393) |
| | Aerial mycelium | Grayish white (390), Powdery, Thin |
| | Diffusible pigment | None |
| Glycerol nitrate agar | Vegetative mycelium | Pale reddish yellow (130) |
| | Reverse | Pale reddish yellow (130) |
| | Aerial mycelium | None |
| | Diffusible pigment | None |
| Glucose asparagine agar (Waksman med. No. 2) | Vegetative mycelium | Yellowish white (393) |
| | Reverse | Yellowish white (393) |
| | Aerial mycelium | White (388), Powdery |
| | Diffusible pigment | None |
| Yeast extract-malt extract agar (ISP med. No. 2) | Vegetative mycelium | Dark grayish brown (123) |
| | Reverse | Dark grayish brown (122) |
| | Aerial mycelium | Pale yellowish pink (6), Powdery |
| | Diffusible pigment | Dull yellow |
| Oatmeal agar (ISP med. No. 3) | Vegetative mycellium | Dull yellow (152) |
| | Reverse | Dull yellow (152) |
| | Aerial mycelium | Pale yellowish pink (6), Powdery |
| | Diffusible pigment | Light grayish blue (281) |
| Inorganic salts-starch agar (ISP med. No. 4) | Vegetative mycelium | Yellowish white (393) |
| | Reverse | Yellowish white (393) |
| | Aerial mycelium | None |
| | Diffusible pigment | None |
| Glycerol asparagine agar (ISP med. No. 5) | Vegetative mycelium | Light grayish brown (111) |
| | Reverse | Light grayish brown (111) |
| | Aerial mycelium | White (388), Powdery, Thin |
| | Diffusible pigment | None |

| Table 1. Cultural characteristics of strain AA9966 | | |
|---|---|---|
| Tyrosine agar (ISP med. No. 7) | Vegetative mycelium | Light grayish brown (109) |
| | Reverse | Light grayish brown (109) |
| | Aerial mycelium | None |
| | Diffusible pigment | None |
| Nutrient agar (Waksman med. No. 14) | Vegetative mycelium | Dark yellowish brown (106) |
| | Reverse | Dark yellowish brown (106) |
| | Aerial mycelium | None |
| | Diffusible pigment | None |
| Yeast starch agar | Vegetative mycelium | Dark brown (104) |
| | Reverse | Dark grayish brown (122) |
| | Aerial mycelium | Pale yellowish pink (6), Powdery, Good |
| | Diffusible pigment | Grayish yellow (156) |
| Gauze's agar | Vegetative mycelium | Grayish yellow (157) |
| | Reverse | Grayish yellow (157) |
| | Aerial mycelium | White (388), Powdery, Scant |
| | Diffusible pigment | None |
| Oatmeal-yeast extract agar | Vegetative mycelium | Dark grayish brown (123) |
| | Reverse | Dark grayish brown (123) |
| | Aerial mycelium | Pale yellowish pink (6), Powdery |
| | Diffusible pigment | Light grayish blue (281) |
| Bennett's agar (Waksman med. 30) | Vegetative mycelium | Dark yellowish brown (106) |
| | Reverse | Dark yellowish brown (106) |
| | Aerial mycelium | Pale pink (4), Powdery, Good |
| | Diffusible pigment | None |
| Peptone-corn agar | Vegetative mycelium | Dark yellowish brown (106) |
| | Reverse | Dark yellowish brown (106) |
| | Aerial mycelium | None |
| | Diffusible pigment | None |

Table 2. Physiological characteristics of strain AA9966

| Test | Result |
|------|--------|
| Starch hydrolysis (on ISP med. No. 4) | Positive |
| Nitrate reduction (Difco, nitrate broth) | Positive |
| Milk (Difco, 10% skimmed milk)<br>Coagulation<br>Peptonization | Positive<br>Positive |
| Cellulose decomposition (sucrose nitrate solution with a paper strip as the sole carbon source) | Negative<br>(Growth: good) |
| Gelatin liquefaction<br>On plain gelatin<br>On glucose peptone gelatin | Doubtful<br>Doubtful |
| Melanin formation (On ISP med. No. 7) | Negative |
| Temperature range for growth (°C)<br>Optimum temperature (°C)<br>(On Yeast starch agar) | 20 to 44<br>33 to 43 |
| pH range for growth<br>Optimum pH<br>(On Trypticase soy broth, BBL (Div. Becton, Dickinson & Co.) | 5 to 8<br>6 to 7 |

Table 3. Utilization of carbon sources by strain AA9966

| Carbon Source | Growth |
|---------------|--------|
| D-Glucose | + |
| L-Arabinose | + |
| D-Xylose | + |
| Inositol | ++ |
| Mannitol | + |
| D-Fructose | + |
| L-Rhamnose | + |
| Sucrose | + |
| Raffinose | + |

+: Weak positive, ++: Strong positive
(ISP med. No. 9, 37 °C for 21 days)

3. Chemotaxonomy.

Cell wall composition is analyzed by the method of H.A. Lechevalier and M.P. Lechevalier in "A Critical evaluation of the genera of aerobic actinomycetes," The Actinomycetales (H. Prauser, ed.), pp. 393-405. Jena, Gustav Fischer Verlag, 1970., using thin layer chromatography sheets as described by J.L. Staneck and G.D. Roberts in "Simplified Approach to Identification of Aerobic Actinomycetes by Thin-layer Chromatography," Appl. Microbiol. 28:226-231, 1974. Phospholipid and mycolate composition are determined by the methods of M.P. Lechevalier, C. Debievre and H. Lechevalier in "Chemotaxonomy of Aerobic Actinomycetes: Phospholipid Composition," Biochem. Syst. Ecol. 5: 249-260, 1977 and D.E. Minnikin, L. Alshamaony and M. Goodfellow in "Differentiation of Mycobacterium, Nocardia and Related Taxa by Thin-layer Chromatographic Analysis of Whole-organism Methanolysates," J. Gen. Microbiol. 88: 200-204, 1975, respectively. Menaquinone is analyzed by the procedure of M.D. Collins, H.N. Shah, and D.E. Minnikin in "A Note on the Separation of Natural Mixtures of Bacterial Menaquinones Using Reverse-phase Thin-layer Chromatography," J. Appl. Bacteriol. 48: 277-282, 1980. Fatty acid is determined by the method of K. Suzuki and K. Komagata in "Taxonomic Significance of Cellular Fatty Acid Composition in Some Coryneform Bacteria," Int. J. Syst. Bacteriol. 33: 188-200, 1983.

Whole cell hydrolysates contain meso- diaminopimelic acid, galactose, glucose, mannose and madurose. Therefore, cell wall type is IIIB. Major phospholoids include diphosphatidylglycerol, phosphatidylethanolamine, phosphatidylinositol and unknown glucosamine containing-phospholipids. But phosphatidylglycerol, phosphatidylcholine and phosphatidylmethylethanolamine are not detected. Therefore, phospholipid type is PIV type.

Mycolic acids are not detected. Analysis of the menaquinone composition reveal 47% MK-9(H$_4$), 22% MK-9(H$_2$), 13% MK-9(H$_6$), 11% MK-9(H$_8$), 5% MK-9(H$_0$), and 2% MK-9(H$_{10}$). Fatty acid composition is shown in Table 4.

The morphological and chemotaxonomic characteristics are consistent with the assignment of strain AA9966 to the genus Microbispora Nonomura and Ohara 1957. Thus, strain AA9966 is identified as Microbispora sp. AA9966.

| Table 4. Fatty acid composition of strain AA9966 | | | | | | |
|---|---|---|---|---|---|---|
| Fatty acid composition (%) | | | | | | |
| Straight chain | | | | | | |
| 14:0 | 15:0 | 16:0 | 17:0 | 18:0 | | |
| 1 | 5 | 11 | 8 | 2 | | |
| Branched chain | | | | | | |
| i-15 | i-16 | i-17 | i-18 | a-15 | a-17 | |
| 3 | 25 | 2 | 1 | 1 | 8 | |
| Unsaturated chain | | | | | | |
| 16:1$^9$ | i-18:1$^9$ | 10Me16 | 10Me17 | 10Me18 | i-20H16 | 20H16 |
| 3 | 1 | 7 | 15 | 3 | 3 | 1 |

Antibiotic Production

The antitumor antibiotic complex BU-4803T containing the A$_1$, A$_2$ and B components is produced by cultivating strain AA9966 or a mutant thereof under submerged conditions in an aqueous nutrient medium. The producing organism is grown in a nutrient medium containing an assimilable carbon source, for example an assimilable carbohydrate. Examples of suitable carbon sources include glucose, cerelose, soluble starch and glycerol. The nutrient medium should also contain an assimilable nitrogen source such as fish meal, yeast extract or ammonium salts. Inorganic salts such as sodium chloride, potassium chloride, magnesium sulfate, calcium carbonate, phosphates, etc. are added if necessary. Trace elements such as copper, manganese, iron, zinc, etc. are added to the medium if desired, or they may be present as impurities of other constituents of the media. The incubation temperature may be any temperature at which the producing strain is able to grow, e.g. from about 20 °C to about 44 °C, but it is preferable to conduct the fermentation at within the range of about 33 °C to about 43 °C. A neutral pH is preferably employed in the medium and production of the antibiotic is generally carried out for a period of about 4 to 8 days. Ordinarily, optimum production is achieved in about 8 days.

For preparation of relatively small amounts of the antibiotic, shake flask and surface culture can be employed, but for the preparation of larger amounts, submerged aerobic culture in sterile tanks is preferred. When tank fermentation is to be carried out, it is desirable to produce a vegetative inoculum in a nutrient broth by inoculating the broth culture with spores from the organism and when a young active vegetative inoculum has been obtained, transferring the inoculum aseptically to the fermentation tank medium. Further agitation may be provided by a mechanical impeller. Antiform agents such as lard oil or silicone oil may also be added if needed. Production of antibiotic BU-4803T complex in the fermentation medium can be readily followed during the course of fermentation by antimicrobial assays using Bacillus subtilis as the test organism.

It is to be understood that the present invention is not limited to the use of the particular preferred strain AA9966 described above or to organisms fully answering the above descrption. It is especially intended to include other BU-4803T producing strains or mutants of the said organism which can be produced by conventional means such as x-ray radiation, ultraviolet radiation, treatment with nitrogen mustards, phage exposure and the like.

## Isolation of BU-4803T $A_1$, $A_2$ and B

Antibiotics BU-4803T $A_1$, $A_2$ and B may be recovered from the fermentation broth by conventional separation methodologies such as extraction, crystallization and various chromatographic techniques. The following isolation and purification sequence is illustrative.

The fermentation broth is first extracted with a suitable organic solvent such as ethyl acetate. After the solvent is evaporated, the residue is dissolved in methanol and then chromatographed on an adsorption column such as Diaion HP-20 using aqueous acetone as the eluant to provide a crude BU-4803T complex. The complex is chromatographed on a reversed phase silica gel column to provide a mixture of the $A_1$ and $A_2$ components and the B component. The $A_1$ and $A_2$ components may be isolated from the mixture by preparative thin layer chromatography using aqueous methanol as the mobile phase. The isolated $A_1$, $A_2$ and B components may be further purified by Sephadex LH-20 column chromatography.

## Preparation of BU-4803T $C_1$, $C_2$ and D.

BU-4803T $C_1$, $C_2$ and D can be prepared from BU-4803T $A_1$, $A_2$ and B, respectively. BU-4803T $A_1$, $A_2$ and B are stable for at least one month when stored as solid form but are labile when dissolved in dimethyl sulfoxide or methanol or aqueous acetonitrile. Thus, BU-4803T $A_1$ in dimethyl sulfoxide is converted to BU-4803T $C_1$ within two hours at room temperature, and within 24 hours when it is dissolved in methanol or aqueous acetonitrile. Similarly, BU-4803T $A_2$ and B are converted to BU-4803T $C_2$ and D, respectively, when they are stored in the aforementioned organic solvents.

## Biological Properties

In vitro antimicrobial activities of BU-4803T $A_1$, $A_2$, B, $C_1$, $C_2$ and D are determined by the serial two-fold agar dilution method using nutrient agar (pH 7.0) and a 5-$\mu$l suspension containing $10^5$ cells per ml as inoculum of the test organisms. The minimum inhibitory concentrations (MIC) are determined after incubation for 18 hours at 32 °C. BU-4803T $A_1$, $A_2$ and B show inhibitory activity against Gram-positive bacteria with MICs of 0.8 to 12.5 $\mu$g/ml, while BU-4803T $C_1$, $C_2$ and D do not show inhibitory activity up to 100 $\mu$g/ml. The test data are summarized in Table 5.

Table 5. In vitro antimicrobial activity of BU-4803T components.

| Test organism | | MIC ($\mu$g/ml) BU-4803T | | | | | |
| --- | --- | --- | --- | --- | --- | --- | --- |
| | | $A_1$ | $A_2$ | B | $C_1$ | $C_2$ | D |
| Staphylococcus aureus | FDA 209P JC-1 | 0.8 | 1.6 | 0.8 | 100 | >100 | >100 |
| Staphylococcus aureus | Smith | 6.3 | 12.5 | 12.5 | >100 | >100 | >100 |
| Staphylococcus aureus | A15036 | 6.3 | 6.3 | 6.3 | >100 | >100 | >100 |
| Micrococcus luteus | ATCC 9341 | 3.1 | 1.6 | 1.6 | >100 | >100 | >100 |
| Bacillus subtilis | ATCC 6633 | 3.1 | 3.1 | 3.1 | >100 | >100 | >100 |
| Escherichia coli | Juhl | >100 | >100 | >100 | >100 | >100 | >100 |
| Escherichia coli | K12 | >100 | >100 | >100 | >100 | >100 | >100 |
| Escherichia coli | NIHJ JC-2 | >100 | >100 | >100 | >100 | >100 | >100 |
| Klebsiella pneumoniae | ATCC 10031 | >100 | >100 | >100 | >100 | >100 | >100 |
| Citrobacter freundii | GN7391 | >100 | >100 | >100 | >100 | >100 | >100 |
| Salmonella typhi | 901 | >100 | >100 | >100 | >100 | >100 | >100 |
| Pseudomonas aeruginosa | A9843A | >100 | >100 | >100 | >100 | >100 | >100 |

BU-4803T $A_2$, B, $C_1$, $C_2$ and D are also tested for their in vitro cytotoxicity against B16-F10 (murine melanoma) and HCT-116 (human colon carcinoma) cells. B16-F10 cells are grown in Eagle's minimum essential medium (Nissui) supplemented with fetal calf serum (FCS, 10%) and kanamycin (60 $\mu$g/ml), and HCT-116 cells are grown in McCoy's 5A Medium (Gibco) supplemented with FCS (10%), benzylpenicillin (100 U/ml) and streptomycin (100 $\mu$g/ml) at 37 °C under humidified atmosphere in a $CO_2$ incubator. The above exponentially growing cells are harvested, counted and suspended in the culture media at $1.5 \times 10^4$ (B16-F10) and $3.0 \times 10^4$ (HCT-

116) cells/ml. The cells are planted into wells of a 96-well tissue culture plate and the test compounds are added thereto. The plates are incubated for 72 hours at 37 °C and then the cytotoxic activities are colorimetrically determined at 540 nm after staining viable cells with neutral red solution. All components of BU-4803T showed potent cytotoxicity as shown in Table 6.

| Table 6. In vitro cytotoxicity | | |
|---|---|---|
| | $IC_{50}$ ($\mu$g/ml) | |
| | B16-F10 | HCT-116 |
| BU-4803T $A_1$ | 2.4 | 4.1 |
| BU-4803T $A_2$ | 0.8 | 2.7 |
| BU-4803T B | 0.7 | 1.9 |
| BU-4803T $C_1$ | 1.2 | 2.5 |
| BU-4803T $C_2$ | 3.1 | 5.3 |
| BU-4803T D | 2.7 | 5.5 |

The in vivo antitumor activity of BU-4803T components are determined in tumor-bearing mice. $CDF_1$ mice are implanted intraperitoneally with $10^6$ cells of P388 leukemia cells per mouse. The test compounds are administered intraperitoneally to the mice once daily on days 1 to 3 (Q1D x 3). At the end of the experiment the median survival time (MST) is determined and used to calculate the %T/C, which is the ratio of the MST of a treated group and the MST of the control group multiplied by 100. A %T/C value of 125 or greater indicates significant antitumor activity. As shown in Table 7, BU-4803T B, C mixture and D show antitumor activity against P388 leukemia in mice.

| Table 7. In vivo antitumor activity against P388 leukemia. | | | | |
|---|---|---|---|---|
| Dose (mg/kg/day) | T/C (%) | | | |
| | 30 | 10 | 3 | 1 |
| BU-4803T A mixture | | 114 | 123 | 105 |
| BU-4803T B | 109 | 105 | 127 | |
| BU-4803T C mixture | 127 | 127 | 109 | |
| BU-4803T D | | 127 | 109 | 100 |

The invention includes within its scope pharmaceutical compositions containing an effective antimicrobial or tumor-inhibiting amount of an antibiotic of the present invention in combination with an inert pharmaceutically acceptable carrier or diluent. Such compositions may also contain other active antimicrobial or antitumor agents and may be made up in any pharmaceutical form appropriate for the desired route of administration. Examples of such compositions include solid compositions for oral administration such as tablets, capsules, pills, powders and granules, liquid compositions for oral administration such as solutions, suspensions, syrups or elixirs and preparations for parenteral administration such as sterile solutions, suspensions or emulsions. They may also be manufactured in the form of sterile solid compositions which can be dissolved in sterile water, physiological saline or some other sterile injectable medium immediately before use.

For use as an antitumor agent, optimal dosages and regimens for a given mammalian host can be readily ascertained by those skilled in the art. For use as an antimicrobial agent, an antibiotic of the present invention is administered so that the concentration of the active ingredient is greater than the minimum inhibitory concentration for the particular organism being treated. It will, of course, be appreciated that the actual dose used will vary according to the particular composition formulated, the route of administration and the particular situs, host and disease being treated. Many factors that modify the action of the drug will be taken into account including age, weight, sex, diet, time of administration, route of administration, rate of excretion, condition of the patient, drug combinations, reaction sensitivities and severity of the disease.

The following examples are provided for illustrative purposes only and are not intended to limit the scope of the invention in any manner.

Example 1 Fermentation of strain AA9966

(a) Seed culture. A loopful of mature slant culture of Microbispora sp. AA9966 was inoculated into a 500-ml Erlenmeyer flask containing 100 ml of the seed medium.consisting of soluble starch (Nichiden Kogaku Co.) 2%, glucose 0.5%, NZ-case (Humco Scheffield Chemical Co.) 0.3%, yeast extract (Oriental Yeast Co.) 0.2%,fish extract D30X (Banyu Nutrient Co.) 0.5%, and $CaCO_3$ 0.3% (pH 7.0). The seed culture was incubated at 28 °C for four days on a rotary shaker (200 rpm).

(b) Production culture. Aliquot (5 ml) of the seed culture was transferred into a 500-ml Erlenmeyer flask containing 100 ml of the production medium consisting of glucose 0.5%, lactose 2%, soluble starch 2%, cornsteep liquor (oji Corn Starch Co.) 1%, Pharmamedia (Trader's Protein) 1.5%, Ebios (dried yeast, Asahi Brewery Co.) 0.2%, and $CaCO_3$ 0.3% (pH 7.0).

Fermentation was carried out for eight days under the same conditions described above for the seed culture. Antibiotic production in the fermentation broth was monitored by paper disc diffusion assay using Bacillus subtilis PCl219 as the test organism on nutrient agar (pH 6.0). The assay sample was prepared by extracting a 3-ml portion of the whole broth with the same volume of n-BuOH at pH 7.2;the mixture was centrifuged at 3000 rpm for 15 min. and the solvent layer was used in the assay. Antibiotic production reached the maximum potency (ca. 100 µg/ml) after eight days of fermentation.

Example 2 Isolation of BU-4803T $A_1$, $A_2$ and B

The harvested fermentation broth (5 liters) was extracted with ethyl acetate (5 liters) and the extract was concentrated in vacuo to dryness (3.3 g). The extract (1.3 g) was dissolved in methanol (30 ml) and charged on a column of Diaion HP-20 (40 mm i.d. x 150 mm, Mitsubishi Chemical Industries Ltd.). The resin was washed with 50% aqueous methanol (0.5 liter) and then eluted with 80% aqueous acetone (0.5 liter). Evaporation of the eluate yielded a crude BU-4803T mixture (543 mg).

The BU-4803T mixture (500 mg) was applied on a column of YMC GEL ODS (40 mm i.d. x 450 mm, ODS-AM 120-S50, YMC Co., Ltd), and developed with a mixture of methanol-0.15% $KH_2PO_4$ adjusted to pH 3.5 (70:30). The eluate was fractionated and the fractions were examined by TLC and HPLC (HPLC conditions: Column: Cosmosil 5C18 AR (4.6 mm i.d. x 150 mm, Nacalai Tesque, Inc.); mobile phase: $CH_3CN$-$H_2O$ (43:57); flow rate: 1.2 ml/min.; detection: UV absorption at 254 nm. TLC conditions: plate: TLC plate RP-18 $F_{254S}$ (Merck, Art 15423); solvent: MeOH-$H_2O$ (87.5:12.5). The BU-4803T A mixture was eluted first followed by BU-4803T B. The appropriate fractions of the eluate were pooled and concentrated to aqueous solution. Each solution was extracted with ethyl acetate and the extract was evaporated in vacuo to give BU-4803T A mixture (159.9 mg) and semi-pure BU-4803T B (35.6 mg).

The semi-pure BU-4803T B was re-chromatographed on a column of Sephadex LH-20 (22 mm i.d. x 450 mm, Pharmacia Fine Chemicals AB) eluting with $CH_2Cl_2$-methanol (1:1) to give pure BU-4803T B (31.8 mg). BU-4803 T $A_1$ and $A_2$ were separated from the A mixture (36 mg) by preparative TLC (RP-18 $F_{254S}$, Art. 15389, E. Merck, Darmstadt) using methanol-water (87.5:12.5) as the developing solvent. Two red bands (BU-4803T $A_1$, Rf 0.49; BU-4803T $A_2$, Rf 0.43) were scraped off, and each was extracted with $CH_2Cl_2$ methanol (1:1). Each extract was concentrated and charged on a column of Sephadex LH-20 (20 mm i.d. x 450 mm) eluting with $CH_2Cl_2$-methanol (1:1). Evaporation of the eluate gave pure BU-4803T $A_1$ (17.7 mg) and BU-4803T $A_2$ (5.1 mg).

Example 3 Preparation of BU-4803T $C_1$, $C_2$ and D

BU-4803T B (20 mg) was dissolved in dimethyl sulfoxide (1 ml) and the solution was kept for 15 hours at 4 °C. The solution was charged on a column of YMC D-ODS-5-ST (20 mm i.d. x 150 mm, YMC Co., Ltd.) developed with $CH_3CH$-$H_2O$ (52:48) at flow rate of 10 ml/min. The eluate was monitored by UV absorption at 300 nm, and the appropriate fractions of the eluate (Rt 17 to 20 min.) were pooled and concentrated to dryness. The residual solid was chromatographed on a column of Sephadex LH-20 (20 mm i.d. x 450 mm) eluting with $CH_2Cl_2$-methanol (1:1). Evaporation of the eluate gave pure BU-4803T D (10.1 mg).

Similarly, BU-4803T $C_1$ (9.1 mg) and $C_2$ (3.6 mg) were obtained from BU-4803T $A_1$ (13 mg) and $A_2$ (5 mg), respectively.

## Claims

1. An antibiotic designated BU-4803T A$_1$ having the following physico-chemical properties:

Nature: red powder

M.P. (°C, dec.): >200

Solubility:

    Soluble in: Dimethyl sulfoxide, methanol, acetonitrile, acetone, and methylene chloride

    Insoluble in: Hexane and water

UV $\lambda_{max}$ nm ($E_{1cm}^{1\%}$):

    in MeOH: 244 (222), 278 (267), 433 (92), 509 (58)

    in 0.1 N HCl-MeOH (1:9): 239 (277), 281 (291), 433 (78), 510 (65)

    in 0.1N NaOH-MeOH (1:9): 236 (217), 278 (292), 437 (119), 613 (51), 648 (50)

IR $\nu_{max}$ (KBr) cm$^{-1}$: 3450, 2940, 1700, 1620, 1455, 1405, 1120, 1055 (as shown in Figure 1).

FAB-MS m/z:

    Positive: 1764 (M+H+Na)$^+$

    Negative: 1741 (M+2H-H)$^-$

Elemental Analysis: C 58.20%; H 6.70%; N <0.3%

HPLC Rt (min.): 10.4

TLC Rf: 0.49

$^1$H-NMR (CDCl$_3$): 0.79 (3H, t), 0.87 (3H, t), 0.95 (3H, d), 1.00 (1H, m), 1.04 (3H, d), 1.10 (1H, m), 1.15 (3H, d), 1.29 (3H, d), 1.30 (1H, m), 1.33 (3H, d), 1.34 (2H, m), 1.35 (3H, d), 1.41-1.94 (13H), 1.93 (1H, dd), 1.94 (1H), 1.97 (1H, dd), 2.04 (1H, dt), 2.22 (1H, dt), 2.24-2.38 (2H), 2.24 (3H, s), 2.38 (3H, s), 2.39 (1H, dd), 2.43 (1H, dd), 2.44 (1H), 2.47 (1H, d), 2.64 (1H, d), 2.69 (1H, qui), 3.03 (1H, dd), 3.12 (1H, dt), 3.16 (1H, dt), 3.24-3.33 (2H, dt), 3.34 (1H, d), 3.49 (1H), 3.50-3.60 (2H, dt), 3.64 (1H, dt), 3.68 (1H, dd), 3.72 (1H), 3.73-3.76 (2H), 3.74 (1H), 3.76 (1H), 3.79-3.82 (1H), 3.87 (3H, s), 3.91 (1H, s), 3.95 (3H, s), 3.97 (3H, s), 3.98 (1H), 4.01 (2H, dd), 4.06-4.13 (2H), 4.16 (2H, dd), 4.21 (1H), 4.27 (1H, dd), 4.36 (1H), 4.48 (1H, dd), 4.52 (1H, dd), 4.62 (1H, dd), 4.66 (1H, s), 4.68 (1H, s), 4.94 (1H, d), 5.09 (1H, d), 5.35 (1H, d), 5.43 (1H, d), 5.83 (1H, s), 7.38 (1H, s), 9.69 (1H, s), 12.21 (1H, s), 13.95 (1H, s), 14.96 (1H, s) (as shown in Figure 7)

2. An antibiotic designated BU-4803T A$_2$ having the following physico-chemical properties:

Nature: red powder

M.P. (°C, dec.): >200

Solubility:

    Soluble in: Dimethyl sulfoxide, methanol, acetonitrile, acetone, and methylene chloride

    Insoluble in: Hexane and water

UV $\lambda_{max}$ nm ($E_{1cm}^{1\%}$):

    in MeOH: 240 (261), 279 (276), 432 (86), 510 (68)

    in 0.1 N HCl-MeOH (1:9): 239 (275), 280 (288), 432 (76), 509 (70)

    in 0.1N NaOH-MeOH (1:9): 235 (214), 278 (279), 436 (113), 615 (54), 647 (54)

IR $\nu_{max}$ (KBr) cm$^{-1}$: 3450, 2940, 1700, 1620, 1455, 1405, 1120, 1055 (as shown in Figure 2)

FAB-MS m/z:

    Positive: 1705 (M+Na)$^+$

    Negative: 1683 (M+2H-H)$^-$

HPLC Rt (min.): 11.1

TLC Rf: 0.43

$^1$H-NMR (CDCl$_3$): 0.78 (3H, t), 0.87 (3H, t), 0.95 (3H, d), 0.99 (1H, m), 1.05 (1H, m), 1.14 (3H, d), 1.25-1.34 (5H), 1.29 (3H, d), 1.30 (3H, d), 1.31 (3H, d), 1.44-2.07 (15H), 1.93 (1H), 1.98 (1H), 2.00 (1H), 2.23 (2H), 2.24 (3H, s), 2.26 (1H), 2.39 (1H, dd), 2.43 (1H, dd), 2.44 (1H), 2.47 (1H), 2.63 (1H, d), 2.69 (1H, qui), 3.03 (1H, dd), 3.12 (1H, dt), 3.16 (1H, dt), 3.20-3.30 (3H), 3.36 (1H, d), 3.49 (1H), 3.47-3.55 (2H), 3.68 (1H, dd), 3.72 (1H), 3.74-3.76 (2H), 3.80 (1H), 3.79-3.82 (1H), 3.87 (3H, s), 3.91 (1H, s), 3.93 (1H, s), 3.95 (3H, s), 3.97 (3H, s), 3.99 (1H), 4.02 (3H), 4.02 (1H), 4.06-4.10 (1H), 4.13-4.17 (2H), 4.21 (1H), 4.27 (1H), 4.35 (1H), 4.42 (1H), 4.49 (1H, dd), 4.52 (1H, d), 4.66 (1H), 4.82 (1H), 4.93 (1H, d), 5.34 (1H, d), 5.43 (1H, d), 5.83 (1H, s), 7.38 (1H, s), 9.69 (1H, s), 12.20 (1H, s), 13.94 (1H, s), 14.96 (1H, s) (as shown in Figure 8)

3. An antibiotic herein designated BU-4803T B having the following physico-chemical properties:

Nature: red powder

M.P. (°C, dec.): >200

Solubility:

    Soluble in: Dimethyl sulfoxide, methanol, acetonitrile, acetone, methylene chloride

    Insoluble in: Hexane and water

UV $\lambda_{max}$ nm ($E_{1cm}^{1\%}$):

    in MeOH: 240 (239), 278 (254), 432 (80), 511 (62)

    in 0.1 N HCl-MeOH (1:9): 239 (260), 281 (271), 431 (71), 510 (67)

    in 0.1N NaOH-MeOH (1:9): 235 (211), 278 (272), 436 (111), 614 (51), 647 (51)

IR $\nu_{max}$ (KBr) cm$^{-1}$: 3450, 2940, 1705, 1620, 1460, 1405, 1125, 1060 (as shown in Figure 3)

FAB-MS m/z:

    Positive: 1749 (M+2H+Na)$^+$

    Negative: 1725 (M+2H-H)$^-$

Elemental Analysis: C 58.45%; H 6.48%; N <0;3%

HPLC Rt (min.): 18.0

TLC Rf: 0.39

$^1$H-NMR (CDCl$_3$): 0.77 (3H, t), 0.85 (3H, t), 0.92 (3H, d), 0.93 (3H, d), 0.97 (1H, m), 1.06 (1H, dt), 1.12 (3H, d), 1.27 (3H, d), 1.30 (1H), 1.31 (3H, d), 1.31 (3H, d), 1.32 (2H), 1.45-1.66 (2H), 1.48 (2H), 1.62 (2H), 1.62-1.96 (4H), 1.66 (2H), 1.88 (1H, dt), 1.92 (1H, dd), 1.93 (1H, dd), 1.98 (2H, m), 1.98 (1H, s), 2.18 (2H), 2.20 (2H), 2.22 (3H, s), 2.23 (3H, s), 2.38 (1H, dd), 2.41 (1H, dd), 2.49 (1H), 2.52 (1H), 2.63 (1H, d), 2.67 (1H, qui), 3.02 (1H, dd), 3.10 (1H, dd), 3.13 (1H, dd), 3.24 (2H, dt), 3.37 (1H, d), 3.47-3.56 (2H), 3.54 (1H, s), 3.55 (1H, s), 3.66 (1H, m), 3.73 (1H, m), 3.74 (1H), 3.80 (1H, m), 3.85 (3H, s), 3.93 (3H, s), 3.95 (1H), 3.95 (3H, s), 3.96 (1H, d), 4.00 (1H, d), 4.08-4.23 (3H, m), 4.25 (1H, dt), 4.25 (1H, dt), 4.37 (1H, s), 4.45 (1H, d), 4.51 (1H, d), 4.66 (1H, s), 4.80 (1H, s), 4.92 (1H, d), 4.92 (1H, d), 5.32 (1H, d), 5.40 (1H, d), 5.81 (1H, s), 7.37 (1H, s), 9.69 (1H, s), 12.20 (1H, s), 13.94 (1H, s), 14.96 (1H, s) (as shown in figure 9)

$^{13}$C-NMR (CDCl$_3$): 210.6 s, 210.6 s, 203.5 s, 195.5 s, 187.8 s, 184.8 s, 163.6 s, 158.3 s, 156.8 s, 153.2 s, 151.8 s, 150.6 s, 147.7 s, 139.4 s, 138.5 s, 135.4 s, 125.6 s, 124.7 s, 116.3 s, 113.0 s, 112.1 d, 110.5 s, 108.2 s, 107.9 s, 103.2 d, 103.2 d, 98.9 d, 98.9 d, 98.9 d, 98.7 d, 86.6 d, 85.6 s, 85.4 d, 82.7 s, 79.3 s, 78.9 d, 78.8 d, 78.5 s, 77.2 s, 77.2 s, 77.0 d, 76.3 d, 75.4 d, 75.3 d, 75.2 d, 72.7 d, 72.5 d, 70.6 d, 67.8 d, 67.1 d, 67.0 d, 66.7 d, 66.7 d, 65.1 d, 65.0 d, 61.1 q, 60.9 q, 60.8 q, 55.7 d, 44.4 d, 37.0 t, 35.3 t, 34.8 t, 34.3 t, 30.5 t, 30.4 t, 29.5 t, 29.4 t, 27.8 t, 27.8 t, 27.8 t, 25.0 q, 25.0 q, 24.8 t, 24.8 t, 18.1 q, 18.1 q, 18.0 t, 17.9 q, 17.7 q, 16.6 t, 15.1 q, 14.9 q, 14.6 q, 14.6 q

**4.** An antibiotic designated as BU-4803T C$_1$ having the following physico-chemical properties:

Nature: purple powder

M.P. (°C, dec.): >200

Solubility:

    Soluble in: Dimethyl sulfoxide and methylene chloride

    Slightly soluble in: Methanol and acetonitrile

    Insoluble in: Acetone, hexane and water

UV $\lambda_{max}$ nm ($E_{1cm}^{1\%}$):

    in MeOH: 260 (237), 291 (382), 392 (45), 547 (109)

    in 0.1 N HCl-MeOH (1:9): 260 (237), 291 (382), 391 (45), 546 (115)

    in 0.1N NaOH-MeOH (1:9): 292 (238), 457 (117), 482 (130), 679 (61)

IR $\nu_{max}$ (KBr) cm$^{-1}$: 3450, 2940, 1700, 1625, 1455, 1410, 1120, 1055 (as shown in Figure 4)

FAB-MS m/z:

    Positive: 1732 (M+H+Na)$^+$

    Negative: 1709 (M+2H-H)$^-$

Elemental Analysis: C 57.27%; H 6.27%; N <0.3%

HPLC Rt (min.): 9.7

TLC Rf: 0.38

$^1$H-NMR (CDCl$_3$): 0.76 (3H, t), 0.83 (3H, t), 0.93 (3H, d), 0.98 (1H, m), 1.04 (3H, d), 1.07 (1H, m), 1.19 (3H, d), 1.25-1.38 (5H), 1.33 (3H, d), 1.36 (3H, d), 1.36 (3H, d), 1.38-2.08 (18H), 1.91 (1H), 1.97 (1H), 2.02 (1H), 2.15-2.38 (4H), 2.24 (3H, s), 2.38 (3H, s), 2.43 (1H, dd), 2.46 (1H, dd), 2.49 (1H, d), 2.53 (1H, d), 2.79 (1H, qui), 2.95 (1H, d), 3.11-3.24 (3H), 3.24 (1H), 3.30 (1H), 3.52 (1H), 3.52 (1H), 3.54 (1H), 3.62 (1H, dt), 3.75 (1H, d), 3.76 (1H), 3.78-3.90 (3H), 3.98 (1H, d), 3.99 (1H), 4.02 (1H), 4.05 (1H), 4.10-4.22 (3H), 4.12 (3H, s), 4.18 (3H, s), 4.22-4.33 (3H), 4.34 (1H), 4.49 (1H, dd), 4.53 (1H, dd), 4.62 (1H, dd), 4.66 (1H, s), 4.69 (1H, s), 4.93 (1H, d), 5.09 (1H, d), 5.47 (2H, t), 5.86 (1H, s), 7.59 (1H, s), 12.61 (1H, s), 13.27 (1H, s), 14.05 (1H, s) (as shown in Figure 10)

5. An antibiotic desingated as BU-4803T $C_2$ having the following physico-chemical properties:
Nature: purple powder
M.P. (°C, dec.): >200
Solubility:
  Soluble in: Dimethyl sulfoxide and methylene chloride
  Slightly soluble in: Methanol and acetonitrile
  Insoluble in: Acetone, hexane and water
UV $\lambda_{max}$ nm ($E_{1cm}^{1\%}$):
  in MeOH: 261 (257), 292 (392), 394 (47), 552 (102)
  in 0.1 N HCl-MeOH (1:9): 260 (279), 291 (427), 392 (51), 548 (123)
  in 0.1N NaOH-MeOH (1:9): 292 (302), 457 (139), 482 (151), 676 (74)
IR $\nu_{max}$ (KBr) cm$^{-1}$: 3450, 2940, 1695, 1625, 1455, 1410, 1125, 1055 (as shown in Figure 5)
FAB-MS m/z:
  Positive: 1674 (M+H+Na)$^+$
  Negative: 1651 (M+2H-H)$^-$
HPLC Rt (min.): 10.8
TLC Rf: 0.30
$^1$H-NMR (CDCl$_3$): 0.76 (3H, t), 0.84 (3H, t), 0.93 (3H, d), 0.99 (1H, m), 1.09 (1H, m), 1.17 (3H, d), 1.25-1.38 (5H), 1.33 (3H, s), 1.33 (3H, s), 1.34 (3H, s), 1.42-2.08 (17H), 1.92 (1H), 2.15-2.30 (4H), 2.24 (3H, s), 2.41 (1H, dd), 2.46 (1H, dd), 2.49 (1H, d), 2.52 (1H, d), 2.62 (6H, s), 2.78 (1H, qui), 2.88 (1H, d), 3.09-3.30 (6H), 3.49 (1H, dt), 3.52 (1H), 3.53 (1H, dt), 3.75 (1H, d), 3.78-3.86 (3H), 3.95 (1H, d), 4.01 (1H, d), 4.04 (1H, d), 4.10-4.20 (3H), 4.11 (3H, s), 4.17 (3H, s), 4.24-4.30 (3H), 4.34 (1H), 4.43 (1H, dd), 4.50 (1H, d), 4.53 (1H), 4.66 (1H), 4.84 (1H), 4.93 (1H, d), 5.42 (1H, d), 5.46 (1H, d), 5.86 (1H, s), 7.58 (1H, s), 12.63 (1H, s), 13.36 (1H, s), 14.09 (1H, s) (as shown in Figure 11)

6. An antibiotic designated as BU-4803T D having the following physico-chemical properties:
Nature: purple powder
M.P. (°C, dec.): >200
Solubility:
  Soluble in: Dimethyl sulfoxide and methylene chloride
  Slightly soluble in: Methanol and acetonitrile
  Insoluble in: Acetone, hexane and water
UV $\lambda_{max}$ nm ($E_{1cm}^{1\%}$):
  in MeOH: 261 (217), 291 (347), 395 (39), 548 (97)
  in 0.1 N HCl-MeOH (1:9): 260 (243), 291 (383), 392 (45), 545 (113)
  in 0.1N NaOH-MeOH (1:9): 292 (234), 457 (113), 482 (126), 677 (60)
IR $\nu_{max}$ (KBr) cm$^{-1}$: 3450, 2940, 1700, 1625, 1455, 1410, 1125, 1055 (as shown in Figure 6)
FAB-MS m/z:
  Positive: 1717 (M+2H+Na)$^+$
  Negative: 1693 (M+2H-H)$^-$
Elemental Analysis: C 58.32%; H 6.34%; N <0.3%
HPLC Rt (min.): 17.0
TLC Rf: 0.25
$^1$H-NMR (CDCl$_3$): 0.77 (3H, t), 0.84 (3H, t), 0.93 (3H, d), 0.94 (3H, d), 0.99 (1H), 1.07 (1H, m), 1.17 (3H, d), 1.24-1.36 (4H), 1.33 (3H, d), 1.33 (3H, d), 1.34 (3H, d), 1.45-1.86 (6H), 1.88-2.06 (5H), 1.92 (1H), 2.19 (2H), 2.24 (2H), 2.24 (3H, s), 2.24 (3H, s), 2.42 (1H, dd), 2.46 (1H, dd), 2.46 (1H, d), 2.51 (1H, d), 2.78 (1H, qui), 2.88 (1H, brs), 3.11 (1H), 3.13 (1H), 3.18 (1H, dd), 3.25 (2H, m), 3.45 (1H, d), 3.51 (2H), 3.52 (1H, dd), 3.54 (1H, dd), 3.75 (1H, d), 3.76 (1H), 3.78-3.90 (3H), 3.97 (1H, d), 4.01 (1H, d), 4.04 (1H, d), 4.10-4.20 (2H), 4.11 (3H, s), 4.17 (3H, s), 4.24-4.30 (4H), 4.35 (1H), 4.47 (1H, dd), 4.53 (1H, dd), 4.66 (1H), 4.81 (1H), 4.93 (1H, d), 4.93 (1H, d), 5.43 (1H, d), 5.47 (1H, d), 5.86 (1H, s), 7.58 (1H, s), 12.62 (1H, s), 13.37 (1H, s), 14.11 (1H, s) (as shown in Figure 12)
$^{13}$C-NMR (CDCl$_3$): 210.5 s, 210.5 s, 203.7 s, 195.3 s, 183.2 s, 176.1 s, 161.2 s, 158.6 s, 152.2 s, 152.1 s, 151.6 s, 150.2 s, 147.6 s, 145.8 s, 143.2 s, 136.4 s, 125.5 s, 125.3 s, 116.3 s, 116.2 s, 113.7 s, 112.6 d, 112.8 s, 107.5 s, 103.3 d, 103.3 d, 99.0 d, 99.0 d, 98.9 d, 98.4 d, 86.8 d, 86.0 s, 85.7 d, 82.9 s, 79.4 s, 78.9 d, 78.8 d, 78.6 s, 77.2 s, 77.2 s, 76.7 d, 75.8 d, 75.4 d, 75.2 d, 75.0 d, 72.9 d, 72.7 d, 70.8 d, 67.9 d, 67.4 d, 67.4 d, 66.7 d, 66.7 d, 65.4 d, 65.1 d, 62.2 q, 61.6 q, 55.4 d, 44.0 d, 36.7 t, 35.3 t, 34.9 t, 34.1 t, 30.5 t, 30.4 t, 29.5 t, 29.4 t, 28.5 t, 27.8 t, 27.8 t, 25.0 q, 25.0 q, 24.9 t, 24.9 t, 18.1 q, 18.1 q, 18.1 q, 17.8 q, 17.7 t, 16.6 t, 15.1 q, 14.8 q, 14.6 q, 14.6 q.

7. A process for the preparation of an antibiotic designated BU-4803T $A_1$ which comprises cultivating a strain of <u>Microbispora</u> capable of producing said antibiotic in an aqueous medium containing an assimilable source of carbon and nitrogen under aerobic condition, and recovering said antibiotic from the cultured broth.

8. A process for the preparation of an antibiotic designated BU-4803T $A_2$ which comprises cultivating a strain of <u>Microbispora</u> capable of producing said antibiotic in an aqueous medium containing an assimilable source of carbon and nitrogen under aerobic condition, and recovering said antibiotic from the cultured broth.

9. A process for the preparation of an antibiotic designated BU-4803T B which comprises cultivating a strain of <u>Microbispora</u> capable of producing said antibiotic in an aqueous medium containing an assimilable source of carbon and nitrogen under aerobic condition, and recovering said antibiotic from the cultured broth.

10. A biologically pure culture of the microorganism <u>Microbispora</u> strain AA9966 having the identifying characteristics of ATCC 55327 and is capable of producing an antibiotic selected from the group consisting of BU-4803T $A_1$, BU-4803T $A_2$ and BU-4803T B upon cultivation in an aqueous medium containing an assimilable source of carbon and nitrogen.

WAVELENGTH IN MICRONS

FIG.1

EP 0 592 835 A2

## FIG.2

FIG.3

## FIG.4

WAVELENGTH IN MICRONS

WAVENUMBER (cm⁻¹)

PERCENT TRANSMISSION

EP 0 592 835 A2

FIG.5

WAVELENGTH IN MICRONS

PERCENT TRANSMISSION

WAVENUMBER (cm$^{-1}$)

FIG.6

EP 0 592 835 A2

23

FIG.7

FIG.8

EP 0 592 835 A2

FIG.9

EP 0 592 835 A2

FIG.10

FIG.11

FIG.12

EP 0 592 835 A2